# EUROPEAN PATENT APPLICATION

(11) **EP 2 564 844 A2**
(43) Date of publication of application: **06.03.2013**
(21) Application number: 11775290.7
(22) Date of filing: 28.04.2011
(51) Int. Cl.: A61K 31/366, A61K 31/352, A61K 31/585, A61P 19/00

(54) **COMPOSITION FOR TREATING BONE DISEASE COMPRISING A GLYCEOLLIN AS AN ACTIVE INGREDIENT**

(30) Priority: 28.04.2010 KR 20100039533
(71) Applicant: Kyungpook National University Industry- Academic Cooperation Foundation, Daegu 702-701 (KR)
(72) Inventor: CHOI, Je-Yong, Daegu 702-748 (KR); HAN, Min-su, Daegu 701-774 (KR); JEONG, Jae-Hwan, Daegu 700-412 (KR); LIM, Kyung Eun, Daegu 705-031 (KR); KIM, Yong-Hoon, Daegu 702-280 (KR); HWANG, Young-Hyun, Daegu 706-014 (KR); KIM, In-Kyeom, Daegu 706-023 (KR)
(74) Representative: Winkler, Andreas Fritz Ernst
(86) International application number: PCT/KR2011/003154
(87) International publication number: WO 2011/136585

(57) **Abstract**

The present invention relates to a composition for preventing or treating a bone disease, comprising glyceollin as an active ingredient. The active ingredient, glyceollin promotes degradation of the Runx2 protein in osteoblasts and inhibits the mRNA expression of Rankl in osteoblasts to prevent the differentiation of osteoclasts. Furthermore, glyceollin is capable of ameliorating osteoporosis in animal models for osteoporosis. The present composition is a promising drug or food for prevention or treatment of not only osteoporosis but also bone diseases caused by cancer metastatic to the bone.

## Description

### FIELD OF THE INVENTION

The present invention relates to a composition for preventing or treating a bone disease. More specifically, the present invention relates to a pharmaceutical or food composition for preventing, treating or improving a bone disease, comprising glyceollin as an active ingredient.

### DESCRIPTION OF THE RELATED ART

Normal bone remodeling is a homeostasis maintained by bone formation and bone resorption involving interactions between three cell types, chondrocytes, osteoblasts and osteoclasts. In particular, the proliferation and differentiation of osteoblasts is closely correlated with mineralization process. The mutation or the expression decrease of a transcription factor, RUNX2 in osteoblasts results in no occurrence of the mineralization process. RANKL (receptor activator of NF-κB ligand) has been reported to be expressed in osteoblast differentiation and promote the differentiation of osteoclasts. OPG (Osteoprotegerin) has been suggested to be expressed in osteoblasts and inhibit the differentiation of osteoclasts by suppression of RANKL.

The bone supports soft tissues and weights in human body and protects inner organs from exterior shocks by encircling inner organs. In addition to such supporting functions for muscles and internal organs, it has a pivotal function as a reservoir for essential body mineral components such as calcium, phosphorous and magnesium. In this connection, adult bones with terminated growth are not static, and repeatedly undergo a very dynamic and continuous rebuilding process in which new bone formation and bone resorption occur. This process is called as a bone remodeling (Yamaguchi A., et al., Tanpakushitsu Kakusan Koso., 50 (6Suppl): 664-669 (2005)), The bone turnover, a process in which old bones are removed and replaced with new bones, is essential for not only the repair of micro-damage of bones associated with growth and stress but also the maintenance of bone functions (Cohen-Solal M., et al., Therapie., 58 (5): 391-393 (2003)).

Bone remodeling has been reported to involve two types of cells. One of them is a bone-forming osteoblast and another is a bone-resorbing osteoclast. Osteoblasts produce RANKL (receptor activator of nuclear factor-κB ligand) and its decoy receptor, OPG (osteoprotegerin). RANKL binds to RANK (receptor activator of nuclear factor- κB) on osteoclast progenitor cells that are then maturated to osteoclasts, finally leading to induce bone resorption. However, where OPG binds to RANKL to block the binding between RANKL and RANK, the formation of osteoclasts is inhibited and bone-resorption does not occur at undesirable level (Theill LE., et al., Annu Rev Immunol, 20: 795-823 (2002); Wagner EF., et al., Cwrr Opin Genet Dev., 11: 527-532 (2001)).

Osteoporosis is defined as a disease that is characterized by low bone mass and deterioration of bone microstructure due to various causes, contributing to bone fragility and increased risk of fracture, and is a reduced condition of minerals (particularly, calcium) and matrix that are composed of bone. Further, this disease is developed by unbalanced bone remodeling exhibiting osteoclastic activities higher than osteoblastic activities (Iqbal MM., South Med J., 93 (1): 2-18 (2000)). While the inner structure of normal bones has a compact network, the osteoporosis bone shows a widened space between structures and a thinner microarchitecture that becomes susceptible to skeletal fractures (Stephan JJ., et al., Endocr Regul., 37 (4): 225-238 (2003)). Osteoporosis is classified into postmenopausal osteoporosis in which the bone loss (2-3% in a year) rapidly appears upon initiation of menopause and the risk of spine compression and wrist bone fracture is elevated; senile osteoporosis in which the bone loss (0.5-1% in a year) is developed slowly in elder men and women aged more than 70 years and gradual bone loss of hip and spine bones is induced; and secondary osteoporosis developed by diseases regardless of age (*e.g*., endocrine diseases, gastrointestinal diseases and malign tumors), drugs (*e.g.*, adrenal cortical hormones, anticancer chemotherapy, thyroid hormones, anticonvulsants, antiplatelets, methotexate, cyclosporine, GnRH, etc.), alcohol, smoking or accidents (Rosen, CJ., Engl J Med., 353 (6): 595-603, (2005); Davidson M., Clinicain Reviews., 12 (4): 75-82 (2002)).

Since most patients with breast cancer, prostate cancer or multiple myeloma are subjected to bone metastasis (Kozlow W., et al., J Mannary Gland Biol Neoplasia., 10 (2): 169-180 (2005)), their life span has been suggested to be dependent on bone metastasis. In this connection, high mortality of patients having breast cancer or prostate cancer is due to selective bone metastasis of cancer cells. It has been known that the bone metastasis observed in breast cancer cells is almost osteolytic metastasis, and does not affect directly bone but stimulates osteoclasts (Boyde A., et al., Scan Electron Microsc, 4: 1537-1554 (1986)). On the contrary, the bone metastasis found in prostate cancer is an osteoblastic metastasis. The osteolytic or osteoblastic metastasis has been also reported to relate directly to osteolysis. The cancer cells entering bones proliferate in bone-surrounding microenvironments and thus stimulate the activity of osteoclasts or osteoblasts, thereby determining whether the subsequent bone metastasis leads to an osteolytic or osteoblastic phenotype (Choong PF., et al., Clin Orthop RelatRes., 415S: S19-S31 (2003)). The bone metastasis of cancer cells is developed in about 80% of breast cancer patients, and metastatic breast cancer cells activate osteoclasts (Bendre M., at al., Clin Orthop Relat Res., 415(Suppl): S39-S45 (2003); Palmqvist P., et al., J Immunol., 169(6): 3353-3362 (2002)). Activated osteoclasts disrupt the balance of bone-surrounding microenvironments, thereby resulting in not only frequent development of pathological fractures but also bone-related disorders such as leukoerythroblastic anaemia, hypercalcemia, pain, nervecompression syndromes, and so forth (Roodman GD., N Engl J Med., 350:1655-1664(2004)).

Estrogen deficiency, often found in postmenopausal women, has been known to be a main causing factor of osteoporosis in which it affects osteoblasts and osteoclasts to induce bone resorption higher than bone formation. Accordingly, a hormone such as estrogen has been administered into patients for a long time to treat osteoporosis. Unfortunately, numerous reports have indicated that estrogen therapy increases a risk of diseases including breast cancer and endometrial cancer. Recently, there have been attempted diverse researches to develop a plant-derived phytoestrogen having actions identical to estrogen without side effects. As a result, it has been intensively studied whether an isolated candidate has an efficacy on preventing and treating osteoporosis caused by estrogen deficiency in postmenopausal women. For example, it was reported that phytoestrogen has an estrogen activity in bone and cardio-vascular system while an anti-estrogen activity in breast and uterus. All phytoestrogens are a phenolic compound with structural similarity to estrogen, and include isoflavones, lignans and cumestrans as a representative substance. Estrogen activities of phytoestrogens have been known to inhibit bone resorption and to maintain bone density. Many studies have shown that isoflavones originated from soybeans effectively inhibits bone loss in ovariectomized mice. Given that safflower seeds have been utilized in a popular manner to prevent osteoporosis, it could be distinct that phytoestrogens derived from other plants as well as soybean isoflavones also have an activity for bone protection.

As a novel class of phytoalexin, soybean glyceollin with an anti-estrogenic activity is a selective estrogen receptor modulator (SERM) and is synthesized from daidzein. Current research reports have indicated that the anti-estrogenic activity of glyceollin contributes to prevention of breast and ovarian cancer in an effective manner. Based on these results, glyceollin is a good candidate to treat osteoporosis caused by estrogen deficiency in postmenopausal women in the senses that it may replace a hormone therapy inducing harmful side effects such as breast cancer and endometrial cancer.

Throughout this application, various patents and publications are referenced, and citations are provided in parentheses. The disclosure of these patents and publications in their entities are hereby incorporated by references into this application in order to more fully describe this invention and the state of the art to which this invention pertains.

### DETAILED DESCRIPTION OF THIS INVETNION

### Technical Purposes

The present inventors have made intensive researches to develop a novel substance for preventing or treating bone diseases without adverse effects. As a result, we have found that glyceollin as one of phytoalexins extracted from soybean inhibits the differentiation of osteoblasts to osteoclasts in animal cell models and are effective in treating osteoporosis in osteoporosis-induced animal models, demonstrating glyceollin is a promising therapeutics for treating various bone diseases such as osteoporosis.

Accordingly, it is an object of this invention to provide a composition for preventing or treating a bone disease, comprising glyceollin as an active ingredient.

It is another object of this invention to provide a pharmaceutical composition for preventing or treating a bone disease, comprising glyceollin as an active ingredient.

It is still another object of this invention to provide a food composition for improving a bone disease, comprising glyceollin as an active ingredient.

It is further object of this invention to provide a use of glyceollin for manufacturing a composition for preventing or treating a bone disease.

It is still further object of this invention to provide a method for preventing or treating a bone disease, comprising administering to a patient having the bone disease a pharmaceutical composition comprising glyceollin as an active ingredient.

Other objects and advantages of the present invention will become apparent from the detailed description to follow taken in conjugation with the appended claims and drawings.

### Technical Solutions

In one aspect of this invention, there is provided a composition for preventing or treating a bone disease, comprising glyceollin as an active ingredient.

In another aspect of this invention, there is provided a pharmaceutical composition for preventing or treating a bone disease, comprising (a) a therapeutically effective amount of glyceollin; and (b) a pharmaceutically acceptable carrier.

In still another aspect of this invention, there is provided a food composition for improving a bone disease, comprising (a) a bromatologically effective amount of glyceollin; and (b) a bromatologically acceptable carrier.

In further aspect of this invention, there is provided a use of glyceollin for manufacturing a composition for preventing or treating a bone disease.

In still further aspect of this invention, there is provided a method for preventing or treating a bone disease, comprising administering to a patient having the bone disease a pharmaceutical composition comprising glyceollin as an active ingredient.

The present inventors have made intensive researches to develop a novel substance for preventing or treating bone diseases without adverse effects. As a result, we have found that glyceollin as one of phytoalexins extracted from soybean inhibits the differentiation of osteoblasts to osteoclasts in animal cell models and are effective in treating osteoporosis in osteoporosis-induced animal models, demonstrating glyceollin is a promising therapeutics for treating various bone diseases such as osteoporosis.

The active ingredient used in the present invention, "glyceollin" is one of phytoalexins generated in soybean in hypersensitive responses or fungal stresses.

According to a specific example, the glyceollin compound may be isolated or purified by incubating water-immersed soybean with *Aspergillus sp.* microbes, extracting and purifying with conventional purification processes such as chromatography.

The most prevalent form of glyceollin in nature is glyceollin I and its isomers, glyceollin II and III are present in a minute amount (Clinical Cancer Research 2006;12(23) December 1, 2006, 7159-7164, Antiestrogenic Glyceollins Suppress Human Breast and Ovarian Carcinoma Tumorigenesis).

The active ingredient "glyceollin" encompasses glyceollin I, glyceollin II, glyceollin III or their mixture.

According to a preferred embodiment, glyceollin is glyceollin I, glyceollin II, glyceollin III or their mixture, more preferably, glyceollin I.

According to a preferred embodiment, glyceollin inhibits the differentiation of osteoclasts.

Osteoclasts developed in blood cells (hematopoietic stem cells) play a role in bone degradation when calcium in blood is deficient or bone remodeling occurs. The term used herein "bone resorption" refers to bone degradation by secretion of strong acid and proteases by osteoclasts to bone.

According to a preferred embodiment, glyceollin promotes degradation of the Runx2 protein in osteoblasts or preosteoblasts.

According to a preferred embodiment, glyceollin used in the present invention inhibits the mRNA expression of Rankl (receptor-activator of NFκB ligand) in osteoblasts, thereby suppressing the differentiation of osteoclasts.

Consequently, glyceollin used in the present invention decreases a bone remodeling rate or a bone turnover rate by inhibiting the differentiation of osteoblasts or osteoblasts, thereby exhibiting prevention or treatment efficacies on bone diseases.

Furthermore, glyceollin shows prevention or treatment efficacies on bone diseases by inhibition of osteoclast differentiation.

According to a preferred embodiment, the bone disease to be prevented or treated by the present composition includes bone loss or damage due to cancer metastatic to the bone, osteoporosis, osteomalacia, rachitis, osteitis fibrosa, aplastic bone diseases, metabolic bone diseases, osteolysis, leucopenia, bone malformation, hypercalcemia and nerve compression syndrome.

In particular, the present composition is considerably effective in prevention or treatment of osteoporosis.

Osteoporosis may be classified as a primary osteoporosis and a secondary osteoporosis. The primary osteoporosis may be caused by Vitamin D deficiency, insufficient physical exercise, smoking, menopause in women or senility in men. The secondary osteoporosis may be caused even in young people by certain diseases or drugs. In this case, the bone strength becomes decreased and the probability of bone fracture becomes increased depending on severity of and exposure time to diseases or drugs. Such diseases include hyperthyroidism, hyperparathyroidism, Cushing'S Syndrome (hypersecretion disease of andrenocorticotropic hormone), premature menopause, menopause due to artificial operation (oophorectomy), sexual dysfunction, chronic liver diseases (liver cirrhosis), rheumatoid arthritis, chronic kidney dysfunction and gastrectomy, and such drugs include steroids (corticosteroids), anticovulsant drugs (drugs for epilepsy) and heparin.

The present composition exhibits excellent efficacies on prevention and treatment of bone diseases owing to metastasis of cancer cells or tissues to the bone. For example, the cancer cells or tissues include those derived from stomach cancer, lung cancer, breast cancer, ovarian cancer, liver cancer, rectal cancer, bronchogenic cancer, nasopharyngeal cancer, laryngeal cancer, pancreatic cancer, bladder cancer, colon cancer, cervical cancer, endometrial carcinoma, brain cancer, prostate cancer, bone cancer, skin cancer, thyroid cancer, leukemia, non-Hodgkin lymphoma cancer, parathyroid cancer and ureter cancer, but not limited to.

The present composition may be prepared in a pharmaceutical composition. According to a preferred embodiment, the present composition is a pharmaceutical composition comprising (a) a therapeutically effective amount of glyceollin; and (b) a pharmaceutically acceptable carrier. The term used herein "a therapeutically effective amount" means the amount sufficient to accomplish prevention or treatment efficacies of glyceollin on bone diseases.

In the pharmaceutical compositions of this invention, the pharmaceutically acceptable carrier may be conventional one for formulation, including lactose, amylose, dextrose, sucrose, sorbitol, mannitol, starch, cellulose, polyvinylpyrrolidone, water, syrup, methylcellulose, methylhydroxy benzoate, propylhydroxy benzoate, talc, magnesium stearate and mineral oil, but not limited to.

The pharmaceutical compositions of this invention, further may contain wetting agent, sweetening agent, emulsifier, buffer, suspending agent, preservatives, flavors, perfumes, lubricant, stabilizer, or mixtures of these substances. Details of suitable pharmaceutically acceptable carriers and formulations can be found in Remington's Pharmaceutical Sciences (19th ed., 1995), which is incorporated herein by reference.

A pharmaceutical composition of this invention may be administered orally or parenterally, preferably orally. The correct dosage of the pharmaceutical compositions of this invention will be varied according to the particular formulation, the mode of application, age, body weight and sex of the patient, diet, time of administration, condition of the patient, drug combinations, reaction sensitivities and severity of the disease. The general dosage of the pharmaceutical composition of this invention is in the range of 0.001-100 mg/kg based on adult human.

According to the conventional techniques known to those skilled in the art, the pharmaceutical composition comprising the recombinant adenovirus according to the present invention may be formulated with pharmaceutically acceptable carrier and/or vehicle as described above, finally providing several forms a unit dose form and a multi-dose form. Non-limiting examples of the formulations include, but not limited to, a solution, a suspension or an emulsion in oil or aqueous medium, an extract, an elixir, a powder, a granule, a tablet and a capsule, and may further comprise a dispersion agent or a stabilizer.

The present composition may be prepared in a food composition.

According to a preferred embodiment, the present composition comprises (a) a bromatologically effective amount of glyceollin; and (b) a bromatologically acceptable carrier.

The food composition may comprise not only glyceollin as active ingredients but also conventional additives for preparing food compositions, e.g., proteins, carbohydrates, lipids, nutritive substances and flavors. Non-limiting examples of natural carbohydrates include, but not limited to, monosaccharide (e.g., glucose and fructose), disaccharide (e.g., maltose and sucrose), oligosaccharide, polysaccharide (e.g., dextrin and cyclodextrin) and sugar alcohol (e.g., xylitol, sorbitol and erythritol). Non-limiting examples of flavors include, but not limited to, natural flavors (e.g., thaumatin and extract of Stevia) and synthetic flavors (e.g., saccharin and aspartame). For example, where the food composition of this invention is provided as a drink, it may further comprise citric acid, liquid fructose, sucrose, glucose, acetic acid, malic acid, fruit juice, or extract from *Eucommia ulmoides,* a jujube or *Glycyrrhiza glabra.*

### Advantageous Effects

The features and advantages of this invention are will be summarized as follows:
(i) The present invention provides a composition for preventing or treating a bone disease, comprising glyceollin as an active ingredient.
(ii) The active ingredient, glyceollin promotes degradation of the Runx2 protein in osteoblasts and inhibits the mRNA expression of Rankl in osteoblasts to prevent the differentiation of osteoblasts or osteoblasts. Furthermore, glyceollin is capable of ameliorating osteoporosis developed in animal models administered with high dose of Vitamin D.
(iii) The present composition is effective in prevention or treatment of not only osteoporosis but also bone diseases caused by excessive proliferation and activation of osteoclasts,

### BRIEF DESCRIPTION OF THE DRAWINGS

Fig. 1a shows morphology of MC3T3E1 cells which were treated with 20 µM of glyceollin during cellular proliferation. Morphology of glyceollin-treated cells and those of untreated cells were similar.
Fig. 1b represents number of MC3T3E1 cells which were treated with 10 and 20 µM of glyceollin during cellular proliferation. Numbers of glyceollin-treated and untreated cells were similar.
Fig. 1c represents the result of MTT assay for MC3T3-E1 cells which were treated with 10 and 20 µM of glyceollin during cellular proliferation. Numbers of glyceollin-treated and untreated cells were similar.
Fig. 1d represents analysis of Runx2 protein level in MC3T3E1 cells which were treated with 0, 5, 10, 25 and 50 µM of glyceollin during induction of cellular differentiation. As the concentration of glyceollin increased, the level of Runx2 decreased.
Fig. 1e represents analysis of Runx2 mRNA level in MC3T3E1 cells which were treated with 10 and 20 µM of glyceollin during induction of cellular differentiation. The result indicates that the level of Runx2 mRNA is not changed according to the concentration of glyceollin.
Fig. 1f represents analysis of Runx2 protein level in MC3T3E1 cells which were treated with 20 µM of glyceollin and proteasome inhibitor, MG132, and ones treated only with 20 µM of glyceollin. In cells further treated with MG132, decreased Runx2 protein level was recovered.
Fig. 2a represents analysis of Runx2 protein level in osteoblasts isolated from mouse calvarial which were treated with 0, 10 and 20 µM of glyceollin during induction of cellular differentiation. The level of Runx2 decreased as the concentration of glyceollin increased. D21 means 21 days after.
Fig. 2b represents analysis of Runx2 mRNA level in same condition described in Fig. 2a. The result indicates that the level of Runx2 MRNA is not changed according to the concentration of glyceollin. However, mRNA level of osteocalcin, a differentiation marker of osteoblasts, decreased according to the concentration of glyceollin.
Fig. 2c represents analysis of Rankl and Opg mRNA level measured by real-time PCR. Rankl mRNA decreased according to the concentration of glyceollin, whereas Opg mRNA is similar or slightly increased.
Fig. 3a represents the result of ALP staining for osteoblasts isolated from mouse calvarial and then treated with 0, 10 and 20 µM of glyceollin during induction of cellular differentiation. It was confirmed that differentiation of osteoblast was inhibited by observing decrease of ALP staining (7 days after differentiation).
Fig. 3b represents analysis of osteoblasts differentiation by Alizarin Red staining (21 days after differentiation) in same condition described in Fig. 3a. Differentiation was inhibited according to the concentration of glyceollin.
Fig. 3c represents calcium contents of osteoblasts at 21 days after differentiation in same condition described in Fig. 3a. Calcium concentration decreased as the concentration of glyceollin increased.
Fig. 4a represents analysis of differentiation of RAW264.7 cells to osteoclasts by TRAP staining when treated with 0, 5, 10 and 25 µM of glyceollin during induction of differentiation. The result indicates that the differentiation to osteoclasts was inhibited as the concentration of glyceollin increased.
Figs. 5a, 5b, 6a and 6b are showing the protective effect of glyceollin using osteoporosis mouse model obtained by administration of high dose vitamin D.

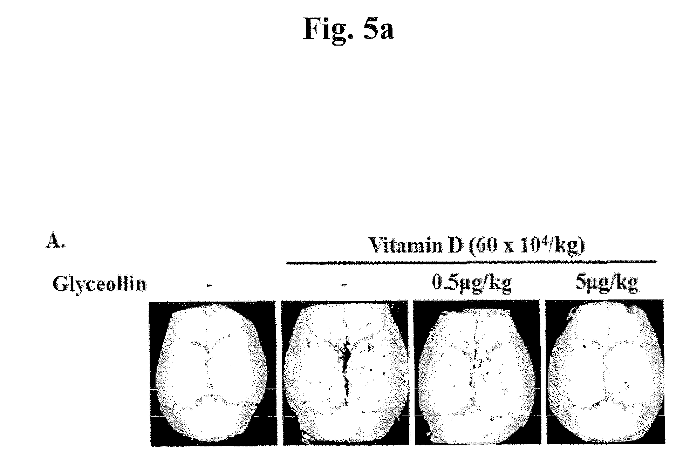

Fig. 5a represents 3-dimensional images of micro CT for mouse model calvarial.

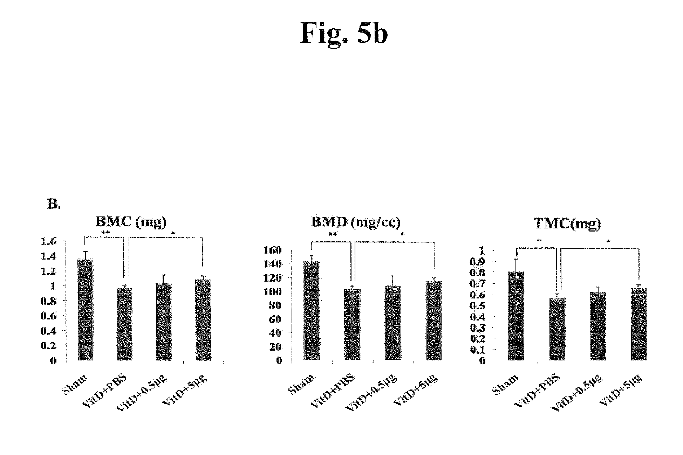

Fig. 5b represents BMC, BMD and TMD analysis executed after CT analysis. The results indicates that osteoporosis of mouse calvarial mediated by high dose vitamin D is recovered by administration of glyceollin.

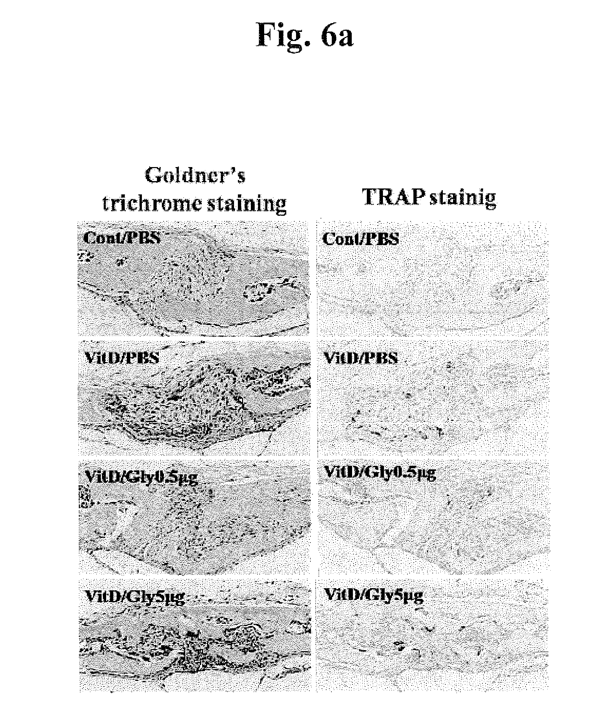

Fig. 6a represents analysis of the effect of glyceollin on generating collagens in osteoporosis mouse model obtained by high dose vitamin D using Trichrome staining method. The result indicates that the collagen generation is increased by glyceollin. In addition, the result of TRAP staining shows that number of osteoclasts is decreased by glyceollin.

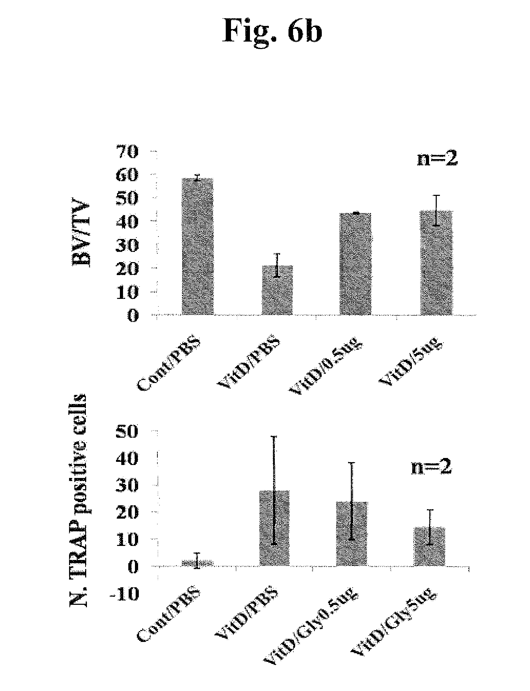

Fig. 6b shows a graph for measurement of Fig. 6a.

The present invention will now be described in further detail by examples. It would be obvious to those skilled in the art that these examples are intended to be more concretely illustrative and the scope of the present invention as set forth in the appended claims is not limited to or by the examples.

### EXAMPLES

### MATERIALS AND METHODS

### 1. Cell culture

Osteoblasts isolated from calvarial of 18.5 days old mice and mouse MC3T3E1 cells were cultured in alpha-MEM containing 10% FBS at 37°C in a humidified atmosphere containing 5% CO₂. To induce differentiation of osteoblasts and MC3T3E1 cells, 50 µg/ml of ascorbic acid and 10mM of beta-glycerophosphate are added to alpha-MEM containing 10% FBS. RAW264.7 (murine monocyte/ macrophage) cells were cultured in D-MEM containing 10% FBS, and RANKL is added to alpha-MEM containing 10% FBS to induce differentiation to osteoclasts.

### 2. Osteoporosis mouse model

Normal mice (C57BL/6) were subcutaneously injected with high dose vitamin D (60 x 10⁴ IU/kg) to obtain osteoporosis mouse model, and monitored during 10 days after first injection. High dose vitamin D activates osteoclasts to reduce mouse bone mass.

### 3. Preparation of glyceollin

Commercially available Glycine max (L.) Merrill (1 kg) was treated with 0.5% of Aspergillus spp. and cultured at 20°C for 3 days, and 210 g of extract containing 1,300 ppm glyceollin was obtained by methanol (5L) solution. The glyceollin-containing extract were dissolved in CH₂Cl₂, and the dissolved fractions were distilled under vacuum and isolated into pure glyceollins I, II, III or mixture thereof by column chromatography using solution of CH₂Cl₂: methanol = 97:3.

### 4. Treatment of glyceollin

MC3T3E1 cells were treated with 0, 5, 10, 25 and 50µM of glyceollin during induction of cellular differentiation. RAW264.7 cells were treated with 0, 5, 10 and 25 µM of glyceollin during induction of cellular differentiation. Osteoblasts isolated from mouse calvarial were treated with 0, 10 and 20 µM of glyceollin during induction of cellular differentiation. Osteoporosis mice induced by high dose vitamin D were daily injected 0, 0.5 and 5 µg/kg of glyceollin for 9 days.

### RESULTS

### 1. Effects of glyceollins on osteoblasts proliferation and differentiation

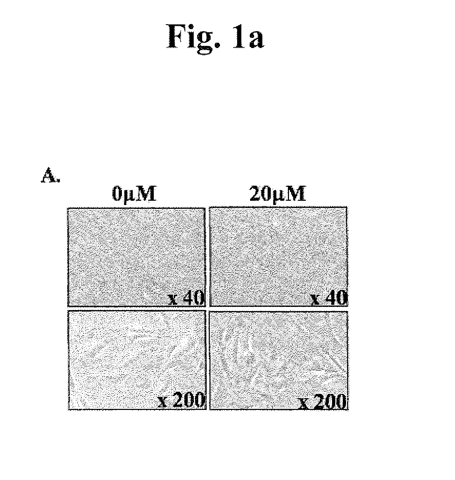

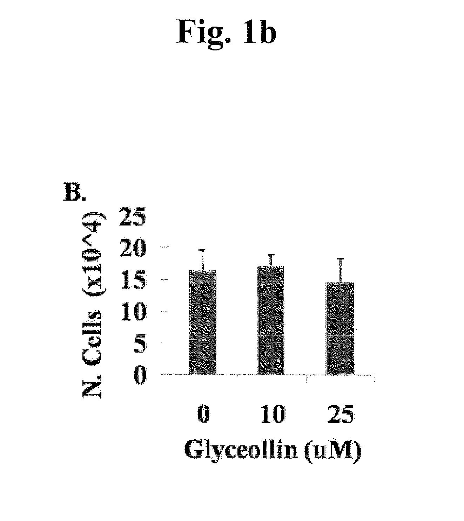

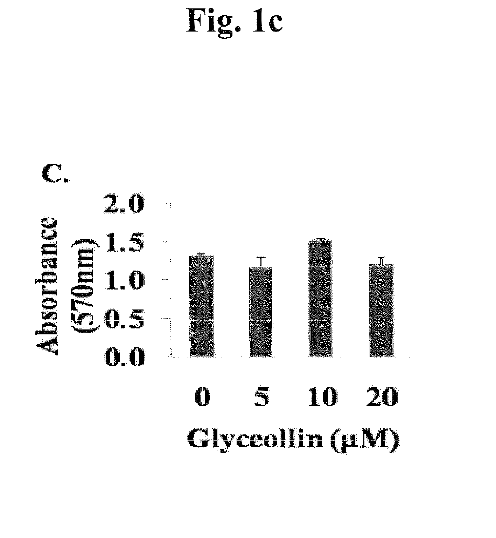

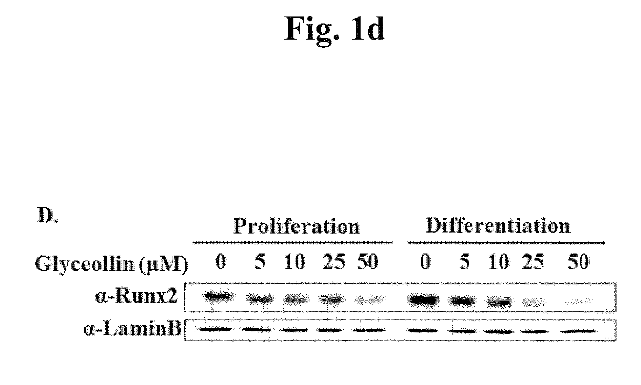

To investigate the function of glyceollin on osteoblasts proliferation, morphology and number of glyceollin-treated MC3T3E1 cells were analyzed and MTT analysis was carried out. As results, no significant difference was observed depending on the treatment of glyceollin (Figs. 1a, 1b and 1c). In addition, in MC3T3E1 cells treated with 0, 5, 10, 25 and 50 µM of glyceollin during induction of cellular proliferation and differentiation, the level of Runx2 protein decreased as the concentration of glyceollin increased (Fig. 1d).

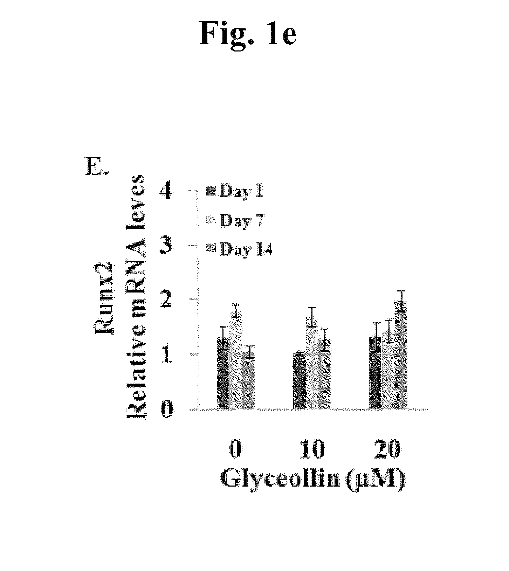

To investigate whether the decrease of Runx2 protein is due to decrease of Runx2 mRNA, mRNA expression patterns were measured after treatment of glyceollin. The result indicates that the expression of Runx2 mRNA is not affected by glyceollin (Fig. 1e),

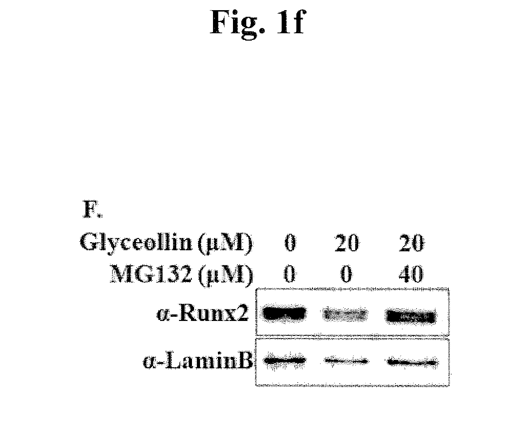

So to elucidate these results were due to the decrease of Runx2 stability through ubiquitin-proteasome degradation, proteasome inhibitor, MG132 were used. The Runx2 protein level decreased by glyceollin was recovered by MG132 (Fig. 1f). These results confirm that inhibitory effect of glyceollin on osteoblasts differentiation is due to its activity to accelerate the degradation of ubiquitin-bound Runx2 in osteoblasts.

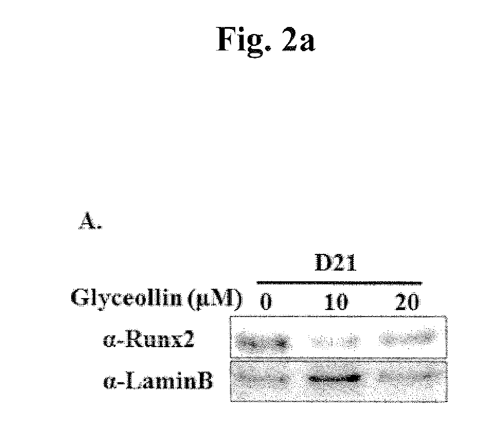

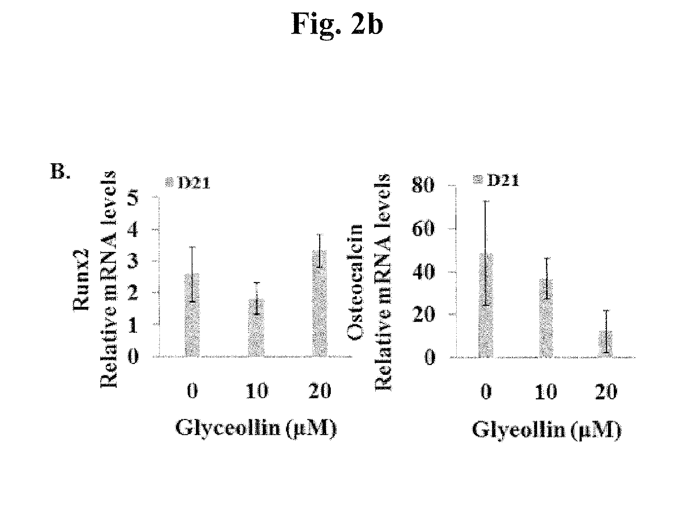

Proteins and mRNAs related to osteoblast differentiation were measured in osteoblasts isolated from mouse calvarial which were treated with 0, 10 and 20 µM of glyceollin. As in MC3T3 cells, the level of Runx2 protein decreased by glyceollin in dose-dependent manner (Fig. 2a), whereas Runx2 mRNA is not affected (Fig. 2b),

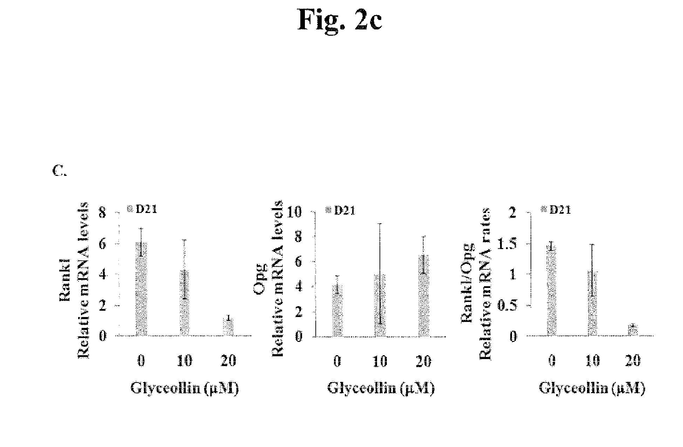

Dose-dependent reduction of osteocalcin mRNA confirmed that glyceollin inhibited differentiation to osteoblast (Fig. 2b). mRNA levels of Rankl (receptor-activator of NF-KB ligand) and Opg (osteoprotegerin) were analyzed by Real-time PCR. As a result, Rankl was down-regulated but its decoy receptor, Opg was not affected or slightly increased by glyceollins (Fig. 2c). Consequently, Rankl/Opg ratio was decreased by glyceollins in osteoblast differentiation (Fig. 2c). These results suggest that glyceollins do not affect osteoblast proliferation, but inhibit differentiation to osteoclast through inducing Runx2 degradation.

### 2. Inhibitory effects of glyceollins in osteoblasts differentiation

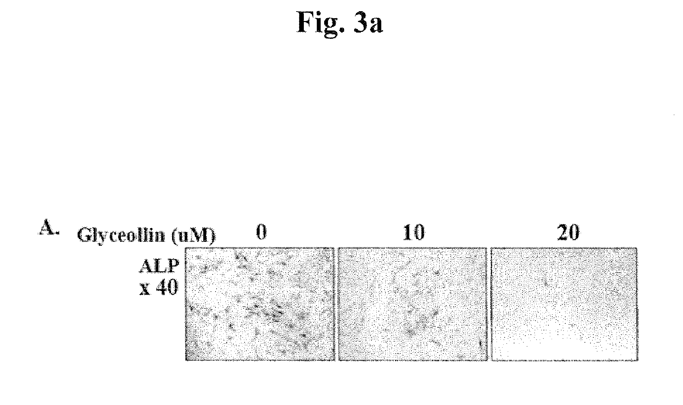

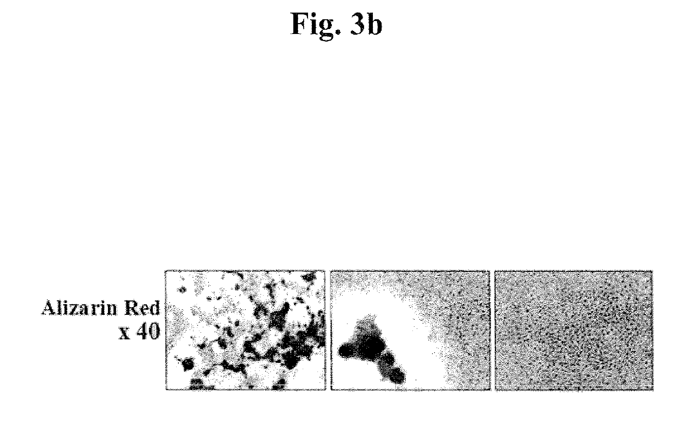

Osteoblasts isolated from mouse calvarial were treated with 0, 10 and 20µM of glyceollin during induction of cellular differentiation, and differentiation was analyzed by ALP staining (7 days after differentiation) and Alizarin Red staining (21 days after differentiation). The differentiation of osteoblast was inhibited by glyceollin in dose-dependent manner (Figs. 3a and 3b)

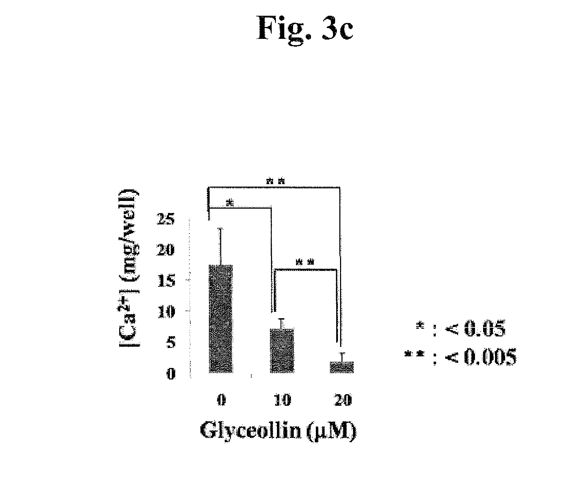

In addition, calcium contents of osteoblasts (21 days after differentiation) were measured and reduction of calcium was observed as glyceollin concentration increased (Fig. 3c). This result coincides with the result of analysis of functional genes involved in osteoblast differentiation.

### 3. Inhibitory effects of glyceollins in osteoclasts differentiation

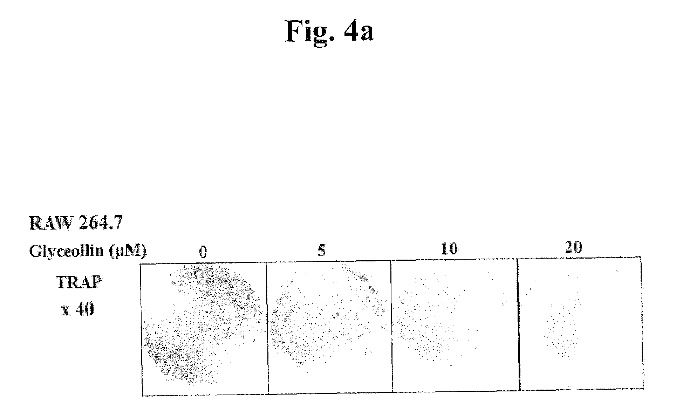

RAW264.7 cells were treated with 0, 5, 10 and 25µM of glyceollin during induction of differentiation, and then TRAP stained 4 days after to analyze osteoclasts. As a result, TRAP stained osteoclasts were reduced by glyceollin in dose-dependent manner (Fig. 4a). This result coincides with the result of analysis of functional genes involved in osteoblast differentiation.

### 4. Protective effects of glyceollin in osteoporosis mouse model

To elucidate *in vivo* inhibitory effect of glyceollin on differentiation of osteoclasts which had been confirmed *in vitro,* osteoporosis mouse model injected with high dose vitamin D one time a day for 3 days. The mice treated with glyceollins one time a day for 9 days were sacrificed at 10 days after first injection and calvarial was observed by micro CT analysis, Trichrome staining and TRAP staining. As a result, bone mineral contents (BMC), bone mineral density (BMD), and tissue mineral contents (TMC) increased by glyceollin in dose-dependent manner (Figs. 5a and 5b). Trichrome staining indicates that collagen generation is increased by glyceollin, and the result of TRAP staining shows the decrease in osteoclasts number by glyceollin (Figs. 6a and 6b).

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A composition for preventing or treating a bone disease, comprising glyceollin as an active ingredient.

2. The composition according to claim 1, wherein the bone disease is bone loss or damage due to cancer metastatic to the bone, osteoporosis, osteomalacia, rachitis, osteitis fibrosa, aplastic bone diseases or metabolic bone diseases.

3. The composition according to claim 1, wherein glyceollin inhibits the differentiation of osteoclasts.

4. The composition according to claim 1, wherein glyceollin promotes degradation of the Runx2 protein in osteoblasts or preosteoblasts.

5. The composition according to claim 1, wherein glyceollin inhibits the mRNA expression of Rankl (receptor-activator of NFκB ligand) in osteoblasts.

6. The composition according to claim 1, wherein glyceollin decreases a bone remodeling rate or a bone turnover rate by inhibiting the differentiation of osteoblasts or osteoclasts.

7. The composition according to claim 1, wherein glyceollin is glyceollin I.

8. A pharmaceutical composition for preventing or treating a bone disease, comprising (a) a therapeutically effective amount of glyceollin; and (b) a pharmaceutically acceptable carrier.

9. A food composition for improving a bone disease, comprising (a) a bromatologically effective amount of glyceollin; and (b) a bromatologically acceptable carrier.

10. A use of glyceollin for manufacturing a composition for preventing or treating a bone disease.

11. A method for preventing or treating a bone disease, comprising administering to a patient having the bone disease a pharmaceutical composition comprising glyceollin as an active ingredient.
